(19) Europäisches Patentamt
European Patent Office
Office européen des brevets

(11) Publication number: **0 409 236 A1**

(12) **EUROPEAN PATENT APPLICATION**

(21) Application number: **90113862.8**

(22) Date of filing: **19.07.90**

(51) Int. Cl.⁵: **C07D 211/32**, A61K 31/445

(30) Priority: **19.07.89 JP 186628/89**

(43) Date of publication of application:
**23.01.91 Bulletin 91/04**

(84) Designated Contracting States:
**AT BE CH DE DK ES FR GB IT LI LU NL SE**

(71) Applicant: **Eisai Co., Ltd.**
**6-10, Koishikawa 4-chome Bunkyo-ku**
**Tokyo(JP)**

(72) Inventor: **Karibe, Norio**
**Eisai Shizanryo, 19-13, Kasuga 4-chome**
**Tsukaba-shi, Ibaraki(JP)**
Inventor: **Sugimoto, Hachiro**
**3073-13, Kashiwadamachi**
**Ushiku-shi, Ibaraki(JP)**
Inventor: **Kaneko, Takeru**
**17-6-1, Matushiro 2-chome**
**Tsukuba-shi, Ibaraki(JP)**
Inventor: **Nakazawa, Takahiro**
**531-1-810, Wada, Fujishiromachi**
**Kitasouma-gun, Ibaraki(JP)**
Inventor: **Ueno, Masataka**
**205, 17-10, Ninomiya 2-chome**
**Tsukaba-shi, Ibaraki(JP)**
Inventor: **Yamatsu, Kiyomi**
**906-313, 8-14, Takezono 1-chome**
**Tsukaba-shi, Ibaraki(JP)**

(74) Representative: **Hansen, Bernd, Dr.**
**Dipl.-Chem. et al**
**Hoffmann, Eitle & Partner Patent- und**
**Rechtsanwälte Arabellastrasse 4e 4**
**D-8000 München 81(DE)**

(54) **Optically active naphthylethanol derivative.**

(57) The optically active naphthylethanol compound is useful in the pharmacological field and defined the formula:

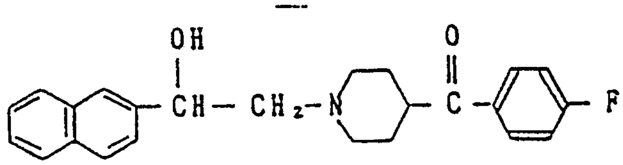

and pharmacologically acceptable salts thereof.

EP 0 409 236 A1

## OPTICALLY ACTIVE NAPHTHYLETHANOL DERIVATIVE

[Field of Industrial Application]

The present invention relates to optically active naphthylethanol derivatives having excellent medicinal effects.

[Prior Art]

Various attempts were made for curing a cerebral vascular disorder with a medicine. Although, for example, cerebral vasodilators and cerebral metabolism activators are used, there is no drastically effective medicine at present. Particularly, there is no medicine effective for cerebral vascular dementia, disturbance of intellectual functions, etc. among various symptoms caused by cerebral vascular disorder at present.

After intensive investigations made for a long time for the purpose of finding new compounds effective in improving symptoms, particularly mental symptoms caused by cerebral vascular disorder under these circumstances, the inventors found cyclic amine derivatives of the following general formula (A) and pharmacologically acceptable salts thereof. See W088/02365 or U.S. patent 4 921 863.

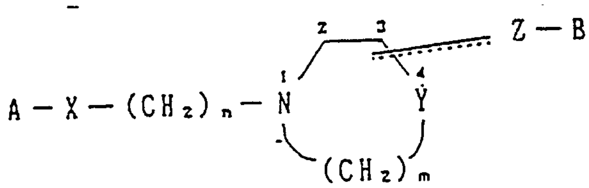

(A)

wherein A represents a substituted or unsubstituted phenyl group, a pyridyl group, a thienyl group, a substituted or unsubstituted naphthyl group, a tetralyl group, a quinolyl group, a benzofuranyl group, a quinazolyl group, a benzothienyl group, a group of the formula:

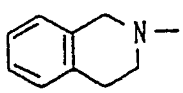 or a group of the formula: 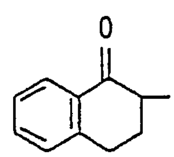 ,

X represents a group of the formula: $-CH_2-$,

a group of the formula: 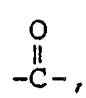, a group of the formula: 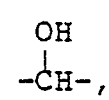, a group of the formula: 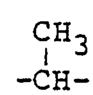

or a group of the formula: 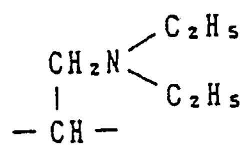 ,

n represents an integer of 0 to 4,
m represents an integer of 1 to 3,
Y represents a carbon or nitrogen atom,
Z represents a group of the formula: $-CH_2-$, a group of the formula:

- -, a group of the formula:

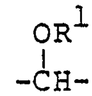

in which $R^1$ represents a hydrogen atom, a lower alkyl group, an acyl group, an arylalkyl group of a heteroarylalkyl group, a group of the formula:

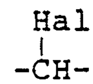

in which Hal represents a halogen atom, a group of the formula: =CH-, a group of the formula:

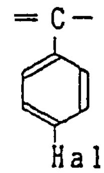

in which Hal represents a halogen atom, a group of the formula:

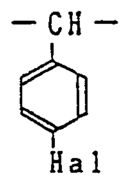

in which Hal represents a halogen atom, or a group of the formula:

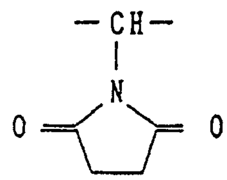 ,

a symbol between Y and Z represents a single or double bond,
a group of the formula: Z - B is bonded to the ring at the 3- or 4-position in the above structural formula, and
B represents a phenyl or naphthyl group which may be substituted with one or two same or different substituents selected from among a halogen, lower alkyl groups and lower alkoxy groups.

The inventors made further detailed investigations of 2-[4-(p-fluorobenzoyl)-1-piperidyl]-1-naphthylethanol of the following chemical structural formula (B) among piperidine derivatives of the above general formula (A):

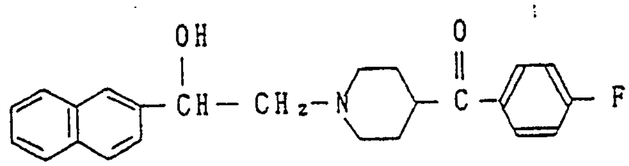 (B)

This compound (B) has an asymmetric carbon atom in its molecule, so that it provides both of d- and ℓ-optical isomers. The inventors investigated these isomers.

Namely, the inventors optically resolved dℓ-2-[4-p-fluorobenzoyl)-1-piperidyl]-1-naphthylethanol produced by, for example, a process described in the above-described W088/02365 to give optically active ℓ- and d-isomers. The medicinal effects of the optically active ℓ- and d-isomers were compared with that of the dℓ-compound to find surprisingly that the medicinal effects of these optically active compounds were far superior to that of the dℓ-compound (racemic compound).

[ Summary of the invention ]

Thus the inventors have found that an optically active naphthylethanol derivative of the following formula (I) or a pharmacologically allowable salt thereof is an excellent agent for improving, curing or preventing mental symptoms caused by a cerebral vascular disorder, and the present invention has been completed on the basis of this finding:

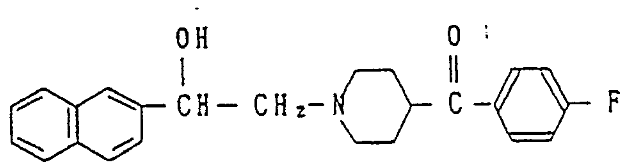

( I )

The optically active compounds are compounds of the following formulae (II) and (III) and pharmacologically allowable salts of them:

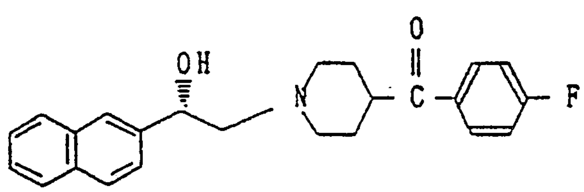

( II )

(S)-(+)-2-[4-(p-fluorobenzoyl)-1-piperidyl]-1-naphthylethanol, and

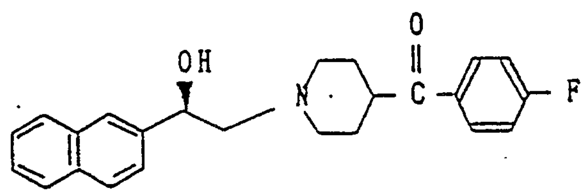

( III )

(R)-(-)-2-[4-(p-fluorobenzoyl)-1-piperidyl-1-naphthylethanol.

The term "pharmacologically allowable salts" as used herein refers to common nonpoisonous salts such as inorganic acid salts, e.g. hydrochloride, hydrobromide, sulfate and phosphate; organic acid salts, e.g. acetate, maleate, tartrate, methanesulfonate, benzenesulfonate and toluenesulfonate; and salts thereof with amino acids, e.g. arginine, aspartic acid and glutamic acid.

The optically active compounds of the present invention can be obtained by resolving the racemic compound by a known process.

Typical processes are as follows:

(1) a process wherein a salt of the racemic compound with an optically active acid such as (L)(-)- or (D)-(+)-tartaric acid, D(+)- or L(-)-malic acid, (R)(-)- or (S)(+)-maleic acid, L(+)-lactic acid, (+)-camphor-10-sulfonic acid or (+)-3-bromocamphor8-sulfonic acid is subjected to fractional crystallization from a suitable solvent,

(2) a process wherein the racemic compound is treated with an optical isomer separating column such as a chiral column, and

(3) a process wherein the racemic compound is converted into an ester thereof with a chiral acid, for example, of the following formula (C) and the ester is isolated as a diastereomer according to column chromatography and deesterified:

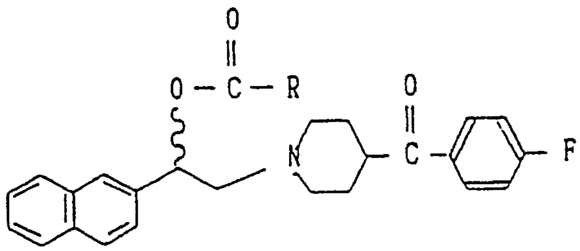

(C)

Preferred esters with chiral acids include esters with alpha-methoxy-alpha-(trifluoromethyl)phenylacetic acid (MTPA).

The following Experimental Examples will illustrate the excellent pharmacological effects of the optically active compounds of the present invention.

Experiment 1

<u>The affinity for dopamine $D_2$ receptor</u>

Rat striatum was used as a membrane source. Striata were homogenized and suspended in Krebs-Tris buffer solution ( 50 mM Tris-HCl(pH 7.4) containing 120 mM NaCl, 5 mM KCl, 2 mM $CaCl_2$ and 1 mM $MgCl_2$).

[$^3H$]-Spiperone (final 0.2 nM) was used as the ligand for $D_2$ receptor and spiperone (final 10 IM) was used for determining non-specific binding. The ligand and various concentrations of the compounds [ the d-isomer and racemate of compound(B)] were mixed with the membrane fraction. The mixture was incubated at room temperature for 30 min and then filtered through a Whatman GF/B filter. The filter was dried and immersed in ACS-II (Amersham) overnight and the radioactivity on the filter was determined with a liquid scintillation counter. Ki values were calculated from the formula, $Ki = IC_{50} / (1-[L] / Kd)$ ( see Josee E. Leysen et al. Biochem. Pharmacol. 27: 307-316).

The results are given in Table 1. The d-isomer has the affinity for $D_2$ receptor 2.9 times as high as that of the racemate.

Table 1

| | Ki (M) |
|---|---|
| d-isomer of compound (B) (compound of Example 1) | $1.7 \times 10^{-7}$ |
| racemate of compound (B) | $4.9 \times 10^{-7}$ |

Experiment 2

<u>Effect on KCN-induced mortality in mice</u>

The effect on the mortality by the intravenous injection of KCN was examined.

Each of the compounds was orally administered to male ddy mice. One hour later 4 mg/kg of KCN

saline solution was intravenously injected. The survival time , i.e. the time from the intravenous injection of KCN to cessation of respiration was recorded.

The results are given in Table 2. The average survival time of control group was 39.1 ± 1.8 sec. The d-isomer of compound(B) at doses of 1, 3 and 10 mg/kg significantly prolonged the survival time.

Table 2

| Effect on the survival time following intravenous injection of KCN in mice | | | |
|---|---|---|---|
| Compound | Dose (mg/kg, p.o.) | n | Survival time (sec) mean ± SEM |
| control group | 5% arabic gum | 10 | 39.1 ± 1.8 |
| d-isomer of compound (B) (compound of Example 1) | 1<br>3<br>10 | 10<br>10<br>10 | 52.4 ± 2.4**<br>50.1 ± 1.6**<br>62.5 ± 4.4** |
| ℓ-isomer of compound (B) (compound of Example 2) | 1<br>3<br>10 | 10<br>10<br>10 | 40.2 ± 1.8<br>43.0 ± 1.8<br>44.8 ± 1.9 |

**: $p < 0.01$ vs control group

Experiment 3

<u>Effect on ischemia-induced neuronal damage in the gerbil hippo-campus</u>

Kirino reported that a brief cerebral ischemia caused the destruction of the pyramidal cells in the CA1 region of the gerbil hippocampus [Brain Res. 239: 57-69 (1982)]. Effect of the compound(B) on this ischemia-induced neuronal damage was examined.

The compounds were intraperitoneally administered to each animal and one hour later 5-min ischemia was produced by clamping both common carotid arteries. One week after the 5-min ischemia gerbils were fixed by transcardiac perfusion of fixative which consisted of 4% formaldehyde in phosphate buffer (pH 7.4). The fixed brain was embedded in paraffin and semi-thin (3 μm) sections were cut at a level of the dorsal hippocampus. The sections were stained with feulgen for cell counting by an image-analyzing computer. Normal pyramidal cells in the area of 0.105 x 1.175 $mm^2$ were counted.

The results are given in Table 3. The neuronal density of pyramidal cells in the CA1 region in the ischemic control group was significantly smaller than that in sham-operated group. The d-isomer of compound(B) protected against the neuronal damage in a dose dependent manner.

Table 3

| Effect on ischemia induced neuronal adamage in the gerbil hippocompus | | | |
|---|---|---|---|
| Compound | Dose (mg/kg, i.p.) | n | neuronal density mean ± SEM |
| Sham group | - | 10 | 34.8 ± 1.2 |
| control group | - | 8 | 4.6 ± 2.4 |
| d-isomer of compound (B) (compound of Example 1) | 1<br>3<br>10 | 7<br>10<br>10 | 9.3 ± 4.6<br>16.5 ± 4.0*<br>34.6 ± 1.1** |
| ℓ-isomer of compound (B) (compound of Example 2) | 3<br>10<br>30 | 6<br>9<br>9 | 1.9 ± 0.5<br>17.2 ± 5.2<br>12.0 ± 3.4 |

*: $p < 0.05$ vs Control
**: $p < 0.01$ vs control

It is apparent from the above-described pharmacological experimental examples that the optically active compound of the present invention, particularly the (S)-(+) compound (compound of Example 1), has a high capability of combining with the dopamine $D_2$ receptor and is excellent in the antianoxic effect and the effect of protecting cells from disorder after ischemia.

Particularly the capability thereof of combining with the dopamine $D_2$ receptor was about three times as high as that of the racemate.

It is thus apparent that the compound of the present invention has a significant pharmacological effect on the central nervous system, particularly a remarkable effect of improving the ischemic cerebral vascular disorder. Therefore, the compound of the present invention is effective in improving, curing or preventing mental symptoms caused by a cerebral vascular disorder such as cerebral stroke, cerebral apoplexy, cerebral infarction, cerebral arteriosclerosis and multiple infarction dementia.

The results of the toxicity tests with rats proved a high safety of the compound of the present invention. Thus the present invention is quite valuable.

The compound of the present invention is used as the medicine for these diseases by oral or parenteral administration. The dose is not particularly limited, since it varies depending on the degree of the symptoms; age, sex, body weight and sensitivity of the patient; method of administration; time and intervals of the administration, and properties, formulation and kind of the medicinal preparation; and kind of the active ingredient. It is usually about 0.1 to 300 mg, preferably about 1 to 100 mg a day for adults. Such a dose of the medicine per day is taken in portions 1 to 4 times a day.

The compound of the present invention is formulated into injections, suppositories, sublingual tablets, tablets or capsules by an ordinary process employed in this technical field.

In the preparation of the injections. If necessary, a pH adjustor, buffering agent, suspending agent, solubilizer, stabilizer, isotonizer, preservative, etc. are added to the main ingredient and formulated into an intravenous, subcutaneous or intramuscular injection by an ordinary method. If necessary, they can be freeze-dried by an ordinary method.

The suspending agents include methylcellulose, Polysorbate 80, hydroxyethylcellulose, acacia, tragacanth powder, sodium carboxymethylcellulose and polyoxyethylene sorbitan monolaurate.

The solubilizers include polyoxyethylenehardened castor oil, Polysorbate 80, nicotinamide, polyoxyethylene sorbitan monolaurate, Macrogol and ethyl ester of castor oil fatty acid.

The stabilizers include sodium sulfite, sodium metasulfite and ether. The preservatives include methyl p-hydroxybenzoate, ethyl p-hydroxybenzoate, sorbic acid, phenol, cresol and chlorocresol.

[Examples]

Typical examples of the present invention will be given below, which by no means limit the present invention.

Example 1

(S)-(+)-2-[4-(p-Fluorobenzoyl)-1-piperidyl]-1-naphthylethanol hydrochloride:

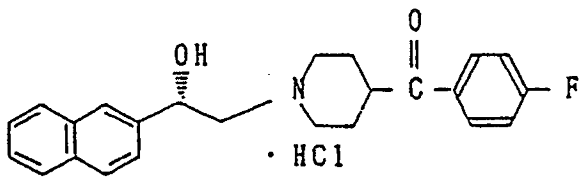

a) Synthesis of 2-{4-[2-(p-fluorophenyl)-1,3-dioxolan-2-yl]-1-piperidyl}-2'-acetonaphthone:

32.6 g of 2-bromo-2'-acetonaphthone, 37.5 g of 2-(p-fluorophenyl)-2-(4-piperidyl)-1,3-dioxolane and 39.5 g of triethylamine were mixed with 500 mℓ of ethanol and the mixture was refluxed for 80 min. Then the solvent was distilled off and dichloromethane was added to the residue. After washing with water followed by drying, the solvent was distilled off and the resultant oily substance was purified through a silica gel column. The resulting crystals were recrystallized from acetone and hexane to give 37.7 g of the intended compound as colorless crystals. Melting point was 112 to 113.2° c.

b) Synthesis of (S)-2-{4-[2-(p-fluorophenyl)-1,3-dioxolan-2-yl]-1-piperidyl}-1-naphthyl-1-ethanol:

2.68 mℓ of a 1 M solution of lithium aluminum hydride in tetrahydrofuran was put in a flask purged with argon. It was cooled to 0 to -5° C and 2.83 mℓ of a 1 M solution of ethanol in tetrahydrofuran was added thereto. Then a solution of 818.9 mg of (R)-1,1-bi-2-naphthol in 4 mℓ of tetrahydrofuran was added thereto. The temperature was elevated to room temperature and the mixture was stirred for 45 min. It was again cooled to -78° C and a solution of 375.8 mg of 2-{4-[2-(p-fluorophenyl)-1,3-dioxolan-2-yl]-1-piperidyl}-2'-acetonaphthone prepared in Example 1-a) in 2 mℓ of tetrahydrofuran was added thereto. After stirring at -78° C for 10 h, 2 mℓ of methanol was added to the mixture and stirred at room temperature for 20 min. 500 ml of ether was added thereto and the resulting mixture was filtered. The solvent was distilled off. Benzene was added resulting mixture was filtered. The solvent was distilled off from the filtrate. Benzene was added to the residue and it was washed with a 3 N aqueous sodium hydroxide solution. After the solvent was distilled off, the residue was purified through a silica gel column to give 282.5 mg of the intended compound as a colorless crystal. The melting point was 133.5 to 134° c and the optical purity was 96.3 %ee, according to a high performance liquid chromatography with chiral cell O J).

c) Synthesis of (S)-(+)-2-[4-(p-fluorobenzoyl)-1-piperidine]-1-naphthylethanol hydrochloride:

30 mℓ of acetone and 30 mℓ of a 1 N aqueous hydrochloric acid solution were added to 282.5 mg of (S)-2-{4-[2-(p-fluorophenyl)-1,3-dioxolan-2-yl]-1-piperidyl}-1-naphthyl-1-ethanol synthesized in Example 1-b) and the resulting mixture was refluxed for 12 h. The solvent was distilled off to give a crystal. It was recrystallized from ethanol to give 215.6 mg of the intended compound as needles.

Melting point: 233.5 to 234° C

$[\alpha]_D^{25}$ : 31.9 (c = 0.75 in ethanol)

Example 2

(R)-(-)-2-[4-(p-Fluorobenzoyl)-1-piperidylj-1-naphthylethanol hydrochloride

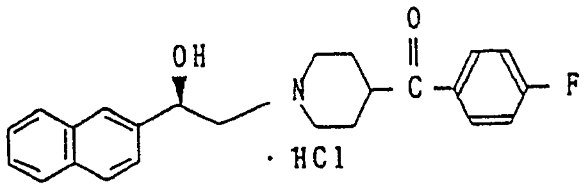

a) Synthesis of (R)-2-{4-[2-(p-fluorophenyl)-1,3-dioxolan-2-yl]-1-piperidyl}-1-naphthyl-1-ethanol:

375.8 mg of 2-{4-[2-p-fluorophenyl)-1,3-dioxolan-2-yl]-1-piperidyl}-2′-acetonaphthone synthesized in Example 1-a) was reacted with (S)-1,1-bi-2-naphthol in the same manner as that of Example 1-b) to give 285 mg of the intended compound.
Melting point: 129 to 130 °C, optical purity: >92.5% e.e.

b) Synthesis of (R)-(-)-2-[4-(p-fluorobenzoyl)-1-piperidyl]-1-naphthylethanol hydrochloride:

112.8 mg of (R)-2-{4-[2-(p-fluorophenyl)-1,3-dioxolan-2-yl}-1-piperidine]-1-naphthyl-1-ethanol was reacted under the same conditions as those of Example 1-c) to give 104.4 mg of the intended compound in the form of colorless needles.
Melting point: 234 to 236.5 °C $[\alpha]_D^{25}$ : -30.8 (c = 0.75 in ethanol.)

**Claims**

1. Optically active 2-[4-(p-fluorobenzoyl)-1-piperidyl]-1-naphthylethanol of the following chemical structural formula:

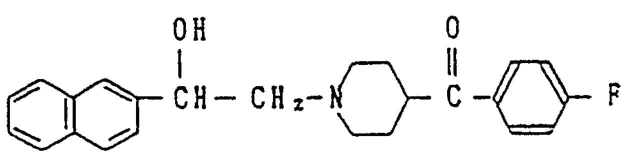

and pharmacologically acceptable salts thereof.
2. An optically active naphthylethanol derivative or a pharmacologically acceptable salt thereof according to Claim 1 which is (S)-(+)-2-[4-(p-fluorobenzoyl)-1-piperidyl]-1-naphthylethanol.
3. An optically active naphthylethanol derivative or a pharmacologically acceptable salt thereof according to Claim 1 which is (R)-(-)-2-[4-(p-fluorobenzoyl)-1-piperidyl]-1-naphthylethanol.
4. An agent for improving, curing or preventing mental symptoms caused by a cerebral vascular disorder which contains, as an active ingredient, optically active 2-[4-(p-fluorobenzoyl-1-piperidyl]-1-naphthylethanol of the following chemical structural formula:

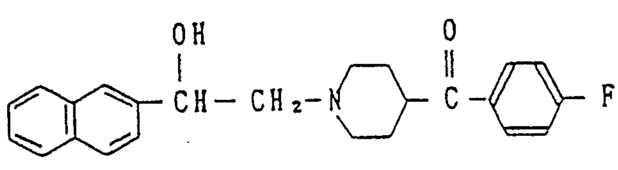

or a pharmacologically acceptable salt thereof.

5. An agent for improving, curing or preventing mental symptoms caused by a cerebral vascular disorder according to Claim 4 wherein the compound is (S)-(+)-2-[4-(p-fluorobenzoyl)-1-piperidyl]-1-naphthylethanol or a pharmacologically acceptable salt thereof.

6. An agent for improving, curing or preventing mental symptoms caused by a cerebral vascular disorder according to Claim 4 wherein the compound is (R)-(-)-2-[4-(p-fluorobenzoyl)-1-piperidyl]-1-naphthylethanol.

7. Use of a compound or a pharmacologically acceptable salt thereof according to claims 1 to 3 for preparing a pharmaceutical composition.

8. Use of a compound or a pharmacologically acceptable salt thereof according to claims 1 to 3 for preparing an agent for improving, curing or preventing mental symptoms caused by cerebral vascular disorder.

European Patent Office

# EUROPEAN SEARCH REPORT

Application Number

EP 90113862.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP - A1 - 0 288 563 (EISAI CO.) * Example 58; claim 25 * | 1,4-8 | C 07 D 211/32 A 61 K 31/445 |
| X,D | & WO-A1-88/02 365 | | |
| X,D | & US-A-4 921 863 | | |
| | ---- | | |

TECHNICAL FIELDS SEARCHED (Int. Cl.5)

C 07 D 211/00

The present search report has been drawn up for all claims

| Place of search | Date of completion of the search | Examiner |
|---|---|---|
| VIENNA | 10-10-1990 | KÖRBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)